# EUROPEAN PATENT APPLICATION

(11) **EP 4 393 436 A1**
(43) Date of publication of application: **03.07.2024**
(21) Application number: 23220734.0
(22) Date of filing: 29.12.2023
(51) Int. Cl.: A61B 18/14, A61B 18/00

(54) **CATHETER WITH FLEXIBLE POLYMER AS OUTER SUPPORT STRUCTURE**

(30) Priority: 30.12.2022 US 202263478059 P; 09.11.2023 US 202318505956
(71) Applicant: Biosense Webster (Israel) Ltd., 2066717 Yokneam (IL)
(72) Inventor: VAN NIEKERK, Pieter Emmelius, Irvine, 92618 (US); EBRAHIMI, Babak, Irvine, 92618 (US); ABBAS, Mohammad, Irvine, 92618 (US); RODRIGUEZ SOTO, Juan, Irvine, 92618 (US)
(74) Representative: Carpmaels & Ransford LLP

(57) **Abstract**

An end effector for a catheter that can include a flexible circuit layer extending along a longitudinal axis from a proximal portion to a distal portion, a framework extending generally parallel to the flexible circuit layer along the longitudinal axis from the proximal to the distal portion, and a flexible polymer layer encapsulating both the framework and the flexible circuit layer. The flexible circuit layer can include a first surface and a second surface opposite the first surface.

## Description

### CROSS REFERENCE TO RELATED APPLICATIONS

This application claims the benefit of priority under 35 U.S.C. § 119 to prior filed U.S. Provisional Patent Application No. 63/478,059, filed December 30, 2022, the entire contents of which is hereby incorporated by reference as if set forth in full herein.

### FIELD

The present invention relates generally to minimally invasive medical devices, and in particular sensing catheters, and further relates to, but not exclusively, cardiac mapping catheters.

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Sources of undesired signals can be located in tissue of an atria or a ventricle. Unwanted signals are conducted elsewhere through heart tissue where they can initiate or continue arrhythmia.

Procedures for treating arrhythmia include surgically disrupting the origin of the signals causing the arrhythmia, as well as disrupting the conducting pathway for such signals. More recently, it has been found that by mapping the electrical properties of the endocardium and the heart volume, and selectively ablating cardiac tissue by application of energy, it is possible to cease or modify the propagation of unwanted electrical signals from one portion of the heart to another. The ablation process destroys the unwanted electrical pathways by formation of non-conducting lesions.

In this two-step procedure, which includes mapping followed by ablation, electrical activity at points in the heart is typically sensed and measured by advancing a catheter containing one or more electrical sensors into the heart and acquiring data at multiple points. These data are then utilized to select the target areas at which ablation is to be performed.

For greater mapping resolution, it is desirable for a mapping catheter to conform closely to the target anatomy. For mapping within an atria or a ventricle (for example, an apex of a ventricle), it is desirable for a catheter to collect larger amounts of data signals within shorter time spans. It is also desirable for such a catheter to be capable of allowing sufficient electrode contact with different tissue surfaces, for example, flat, curved, irregular or nonplanar surface tissue and be collapsible for atraumatic advancement and withdrawal through a patient's vasculature. Existing catheter requires stiff internal structural members to ensure that a predetermined configuration is maintained. The stiffness is a disadvantage during manipulation in the body organ as it can prevent electrodes from contacting the tissue.

### SUMMARY

There is provided, in accordance with an embodiment of the present invention, an end effector for a catheter. The end effector can include a flexible circuit layer extending along a longitudinal axis from a proximal portion to a distal portion, a framework extending generally parallel to the flexible circuit layer along the longitudinal axis from the proximal to the distal portion, and a flexible polymer layer encapsulating both the framework and the flexible circuit layer. The flexible circuit layer can include a first surface and a second surface opposite the first surface.

### BRIEF DESCRIPTION OF THE DRAWINGS

FIG. 1A is an illustration of a perspective view of an end effector, in accordance with an embodiment of the present invention;
FIG. 1B is an illustration of an exploded perspective view of a framework and a flexible circuit layer of an end effector, in accordance with an embodiment of the present invention;
FIG. 1C is an illustration of a side view of an end effector, in accordance with an embodiment of the present invention;
FIG. 1D illustrates yet another variation of the end effector with a different flexible circuit design;
FIG. 2A is an illustration of a top view of the end effector, in accordance with an embodiment of the present invention;
FIG. 2B is an illustration of a top view of the end effector, in accordance with an embodiment of the present invention;
FIG. 2C is an illustration of a top view of the end effector, in accordance with an embodiment of the present invention;
FIG. 2D is a photo of the end effector of Fig. 2B in a typical use scenario with the distal portion of the end effector being curved upward away from the contact surface;
FIG. 2E is a photo of the end effector of Fig. 2C in a typical use scenario with the entire end effector remaining flat;
FIG. 3A is an illustration of a top view of the end effector, in accordance with an embodiment of the present invention;
FIG. 3B is an illustration of a top view of the end effector, in accordance with an embodiment of the present invention;
FIG. 3C is an illustration of a top view of the end effector, in accordance with an embodiment of the present invention;
FIG. 3D is an illustration of a top view of the end effector, in accordance with an embodiment of the present invention;
FIG. 4A is an illustration of a view of a framework and a first side of the flexible circuit layer of an end effector including a magnified view of first electrodes, in accordance with an embodiment of the present invention;
FIG. 4B is an illustration of a view of a framework and a second side of the flexible circuit layer of an end effector including a magnified view of second electrodes, in accordance with an embodiment of the present invention;
FIG. 4C is an illustration of a top view of a framework and a flexible circuit layer of an end effector, in accordance with an embodiment of the present invention;
FIG. 4D is an illustration of a top view of a framework and a flexible circuit layer of an end effector, in accordance with an embodiment of the present invention;
FIG. 5A is an illustration of a perspective view of a framework and a flexible circuit layer having a plurality of coils, in accordance with an embodiment of the present invention;
FIG. 5B is an illustration of a cutaway view of FIG. 5A showing the plurality of coils, in accordance with an embodiment of the present invention; and
FIG. 6 is an illustration of a catheter assembly, in accordance with an embodiment of the present invention.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, reference to tissue, vasculature, or organs of a "patient," "host," "user," and "subject" can be that of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "physician" can include a doctor, surgeon, technician, scientist, or any other individual or delivery instrumentation associated with delivery of a multi-electrode catheter for cardiac mapping of a subject.

In order to achieve the desired stiffness, the mapping resolution, electrode contact with target anatomy, and conformity of the end effector disclosed herein to flat, curved, irregular and/or nonplanar tissue surfaces found in the target anatomy, the end effector has a framework, a flexible circuit layer, and a flexible polymer layer.

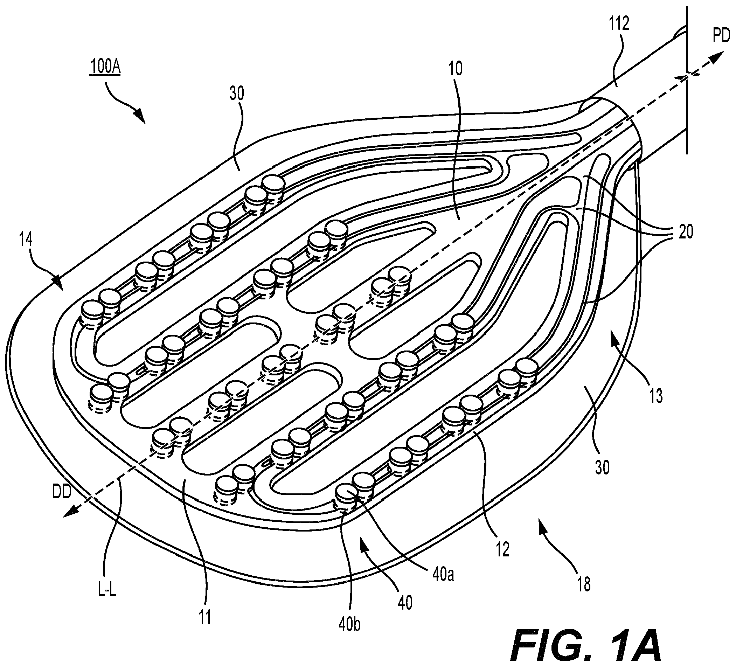

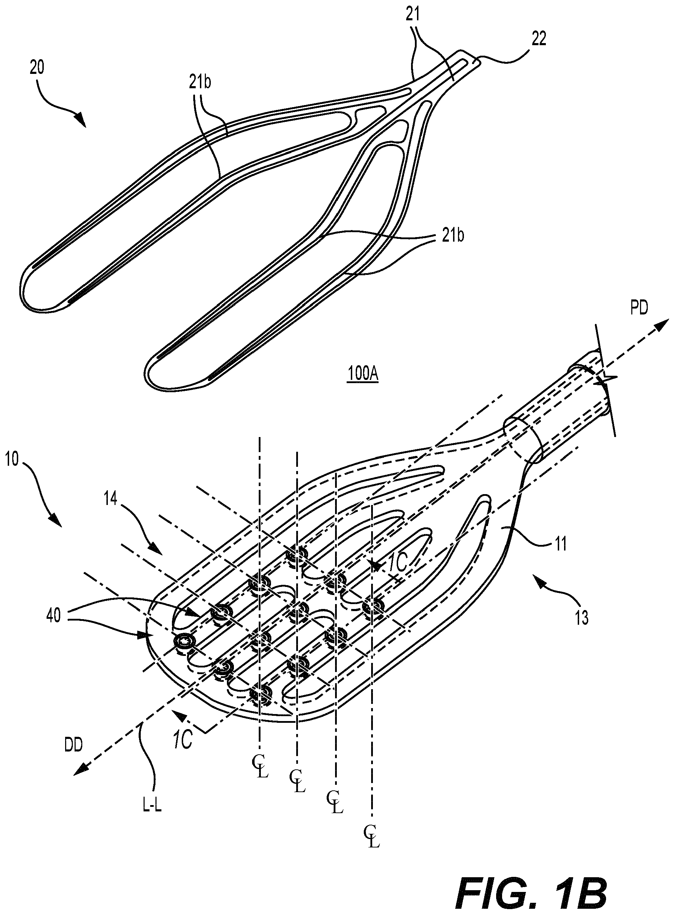

Reference is now made to FIG. 1A showing an end effector 100 for a catheter. The end effector 100 can include a flexible circuit layer 10 extending along a longitudinal axis L-L from a proximal portion 13 to a distal portion 14, a framework 20 coupled to the flexible circuit layer 10 and extending generally parallel to the flexible circuit layer 10 along the longitudinal axis L-L from the proximal 13 to the distal portion 14, and a flexible polymer layer 30 encapsulating both the framework 20 and the flexible circuit layer 10. The flexible circuit layer 10 can include a first surface 11 and a second surface 12 opposite the first surface 11. FIG. 1B shows an exploded view illustrating the flexible circuit layer 10 separated from the framework 20. Importantly, the end effector 100 has an overall flat profile.

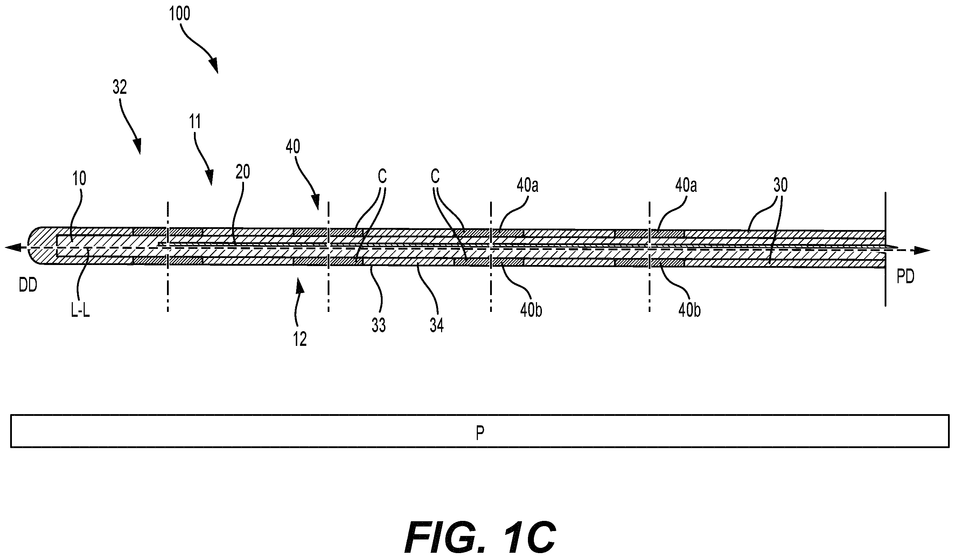

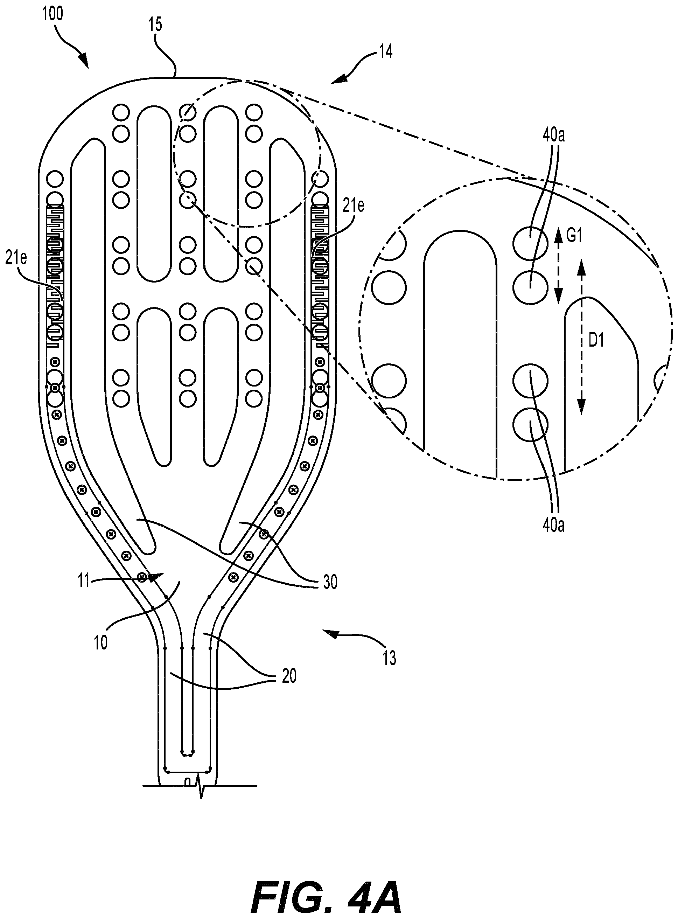

As shown in the example in FIG. 1C, the framework 20 may be embedded within the flexible circuit layer 10. As well, the end effector 100 can further include one or more first electrodes 40a affixed to the first surface 11 of the flexible circuit layer 10 with the one or more first electrodes contact surface C or contact surfaces C exposed through the flexible polymer layer 30 to the ambient environment. In some examples, the contact surfaces C can be exposed by laser cutting through the flexible polymer layer 30. In some examples, the end effector 100 can further include one or more second electrodes 40b affixed to the second surface 12 of the flexible circuit layer 10 with its contact surface C exposed through the flexible polymer layer 30 to the ambient environment. The first electrodes 40a can be axially aligned with the second electrodes 40b to define pairs of opposite facing electrodes 40. Pairs of opposite facing electrodes 40 can be utilized to cancel noise, with one electrode sensing tissue directly and the opposite electrode sensing (far-field signals which typically may include noise), as is appreciated by those skilled in the pertinent art. In some examples, pairs of opposite facing electrodes 40 can be aligned generally parallel to the longitudinal axis L-L. In other examples, pairs of opposite facing electrodes 40 can be aligned generally transverse to the longitudinal axis L-L. More details related to the layout of electrodes 40 are discussed below in relation to FIG. 4A-4B. As shown in FIG. 1C, in some examples, flexible polymer layer 30 can include an outer surface 32, the outer surface having thinner portions 33 surrounding the electrodes 40 and thicker portions at an approximate midpoint between electrodes 40. This can improve the ability of the electrodes 40 to lie flatter against the target anatomy. Alternatively, flexible polymer layer 30 can include an outer surface, the outer surface having thicker portions 34 surrounding the electrodes 40.

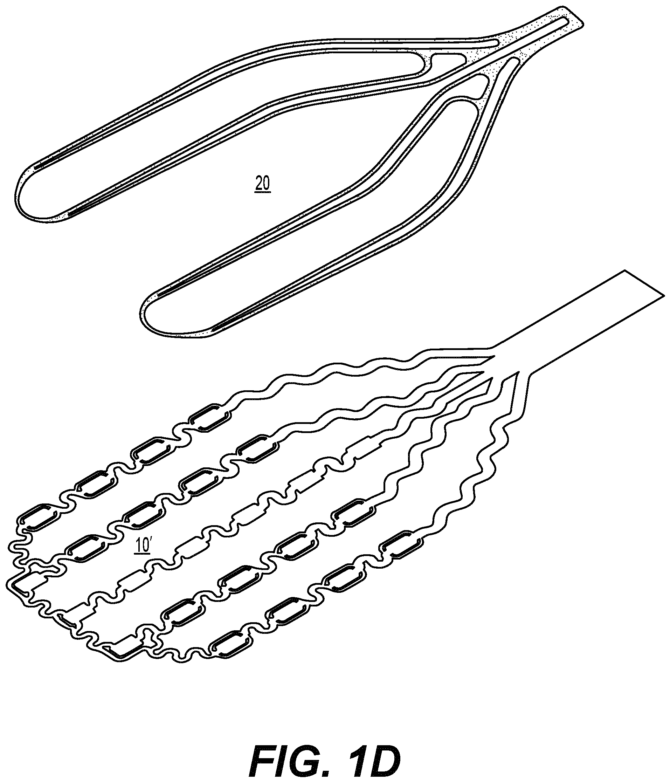

In Fig. 1C, the contact surfaces of the electrodes 40 are configured so that all electrodes 40 physically contact a generally planar surface such as a test jig P during assembly or cardiac tissues in actual use. In Fig. 1D, an alternate design is shown for a framework 20 that can be disposed over the flexible circuit 10' with meandering traces (e.g., sinusoidal-like) between rectangular electrode pads to impart desired stiffness and resilient characteristics for the end effector.

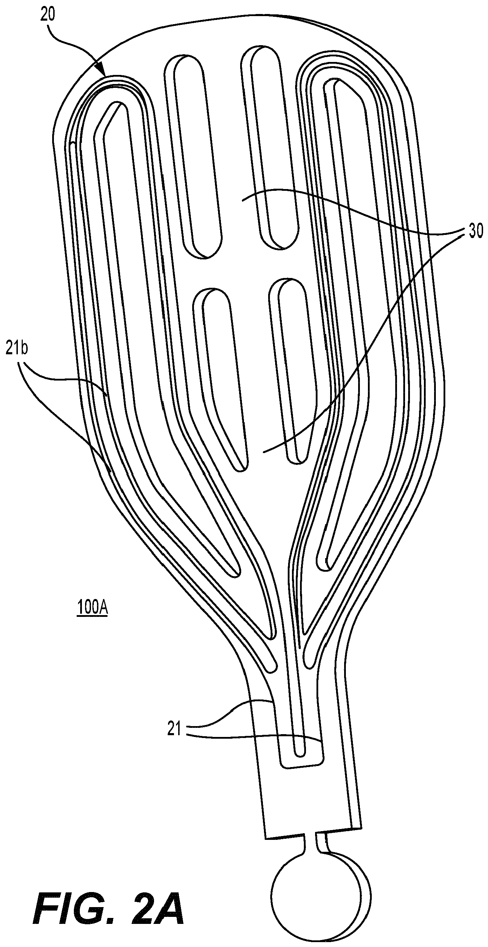

In some examples, such as that shown in FIG. 2A, the framework 20 can be embedded in the flexible polymer layer 30 separate from the flexible circuit 11. This can increase the stiffness of the end effector 100.

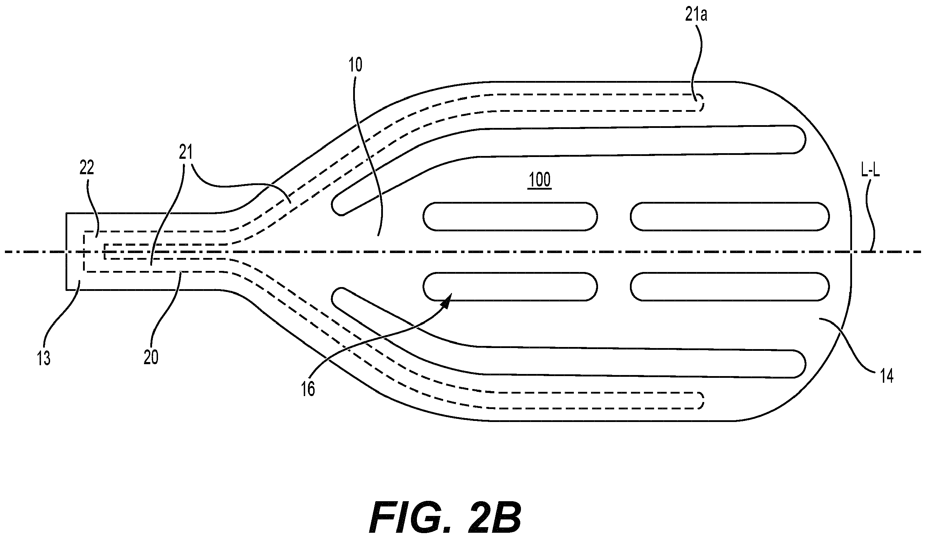

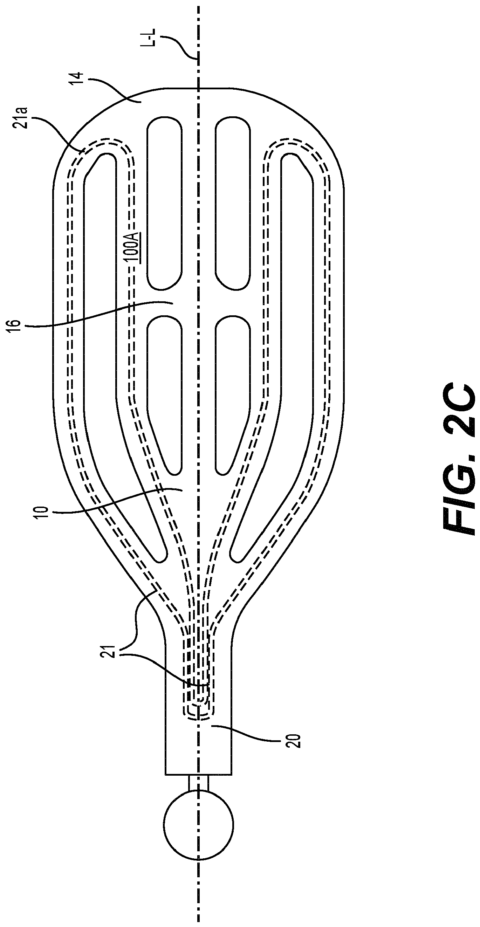

In other examples, such as those shown in FIG. 2B and FIG. 2C, the framework 20 can be embedded in the flexible circuit layer 10. In some examples, the flexible circuit layer 10 defines a planar configuration 18 extending through the longitudinal axis L-L with the framework 20 disposed inside the flexible circuit layer 10 as shown in Fig. 1C.

In other examples, the framework 20 can be unembedded in the flexible circuit layer 10 and coupled thereto only by the flexible polymer layer 30. This can allow the framework 20 to slip relative to the flexible circuit layer 10 and can improve longitudinal flexibility of the end effector 100.

Area 16 can also be formed in one or all of the flexible circuit layer 10, the framework 20, and/or the flexible polymer layer 30. The area 16 can pass through all three layers 10, 20, 30 or just some. The area 16 can be included to increase the ability of the end effector 100 to fold or bend into a reduced delivery configuration to allow the end effector 100 to be passed through a delivery catheter.

In some examples, the flexible circuit layer 10 can be made primarily of polyimide. In other examples, it can be made of any of biocompatible polyimides, glass-reinforced epoxy laminate materials, copper, or graphene, alone or in combination. The flexible circuit layer 10 can include conductive traces. In some examples, the flexible polymer layer 30 can include biocompatible silicone. In other examples, the flexible polymer layer 30 can include biocompatible flexible polymers, thermoelastic polymer materials, and dielectric materials. The flexible polymer layer 30 can optionally be colored to facilitate laser cutting.

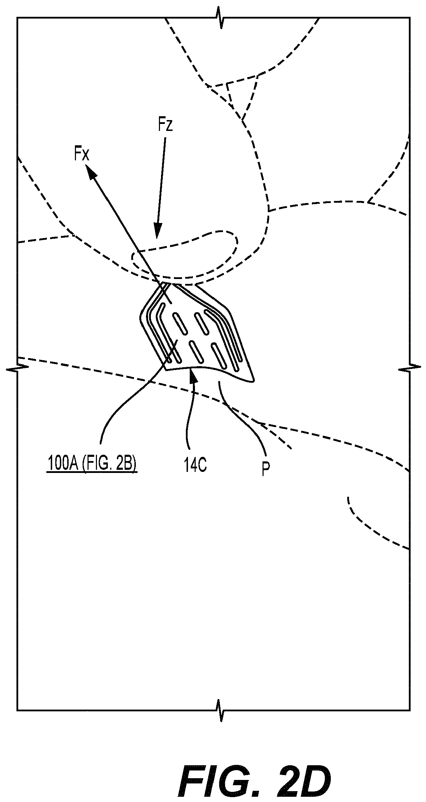

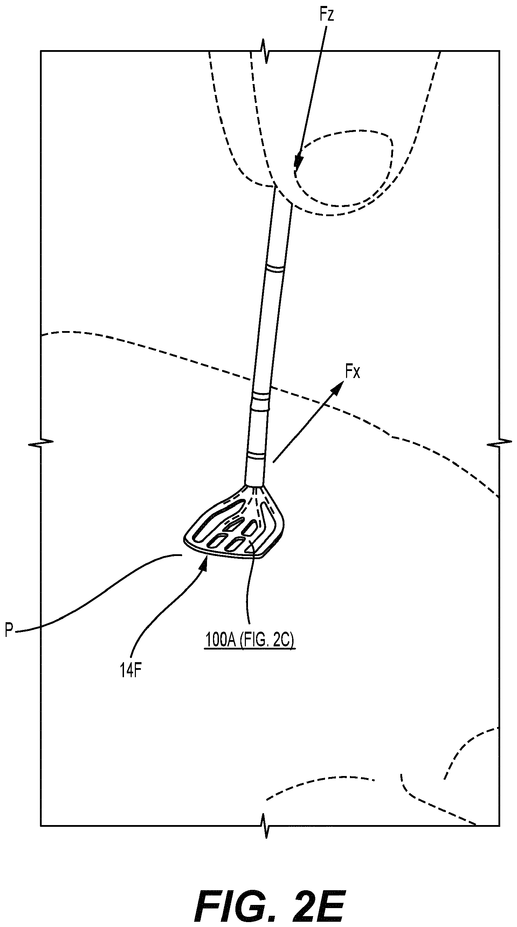

Fig. 2D is a photograph illustrating a desired first configuration for the end effector 100 shown in Fig. 2B for a typical use case in which the end effector against a generally planar surface P. In Fig. 2D, a force applied in the z-direction (Fz) concurrently with a force in the x or y direction (Fx) relative to planar surface P will cause the distal end portion 14 of end effector 100 to curve upward or lifted away from tissue as indicated at 14C. Fig. 2E is a photograph illustrating a desired second configuration of end effector 100A shown in Fig. 2C during typical use case. In Fig. 2E, due to the additional fingers as compared to the single finger of Fig. 2B, a force applied in the z-direction (Fz) concurrently with a force in the x or y direction (Fx) will cause the distal end portion 14 of end effector 100 to remain flat and therefore stay in contact with the tissues to mapped or ablated as indicated by 14F.

FIGs. 2A-FIG. 3D show end effector 100 with various examples of framework 20 embedded inside the polymer layer 30. In the example shown in FIG. 2B, the framework 20 can include a proximal end 22 that divides into two symmetrical side members 21a extending along the longitudinal axis L-L so that no framework 20 is disposed on the area 16 over the longitudinal axis L-L from the proximal portion 13 through the distal portion 14 of the flexible circuit layer 10.

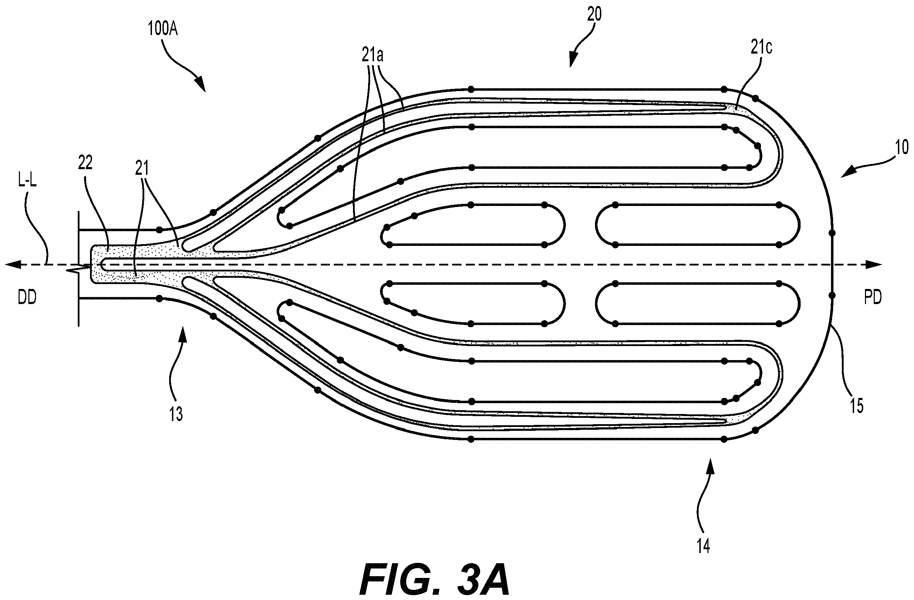

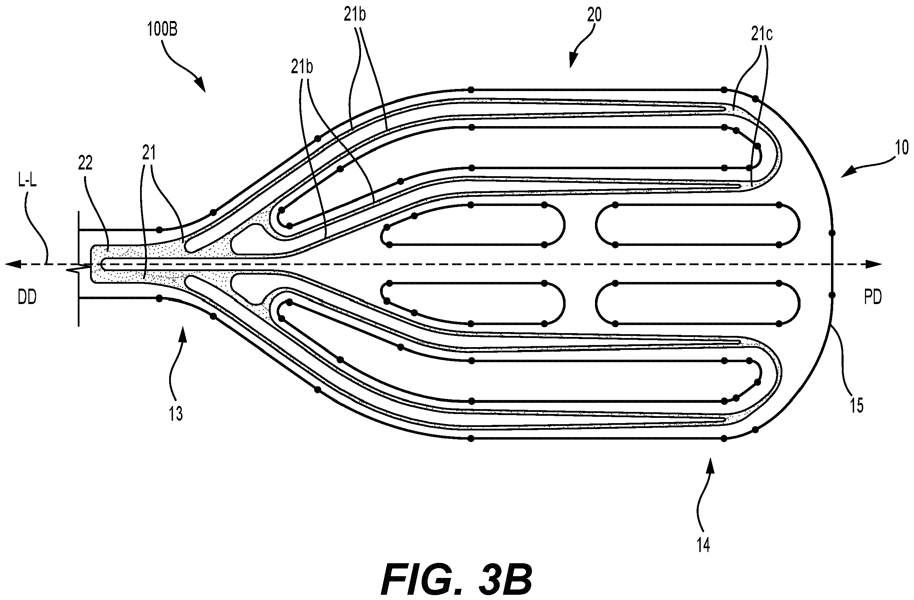

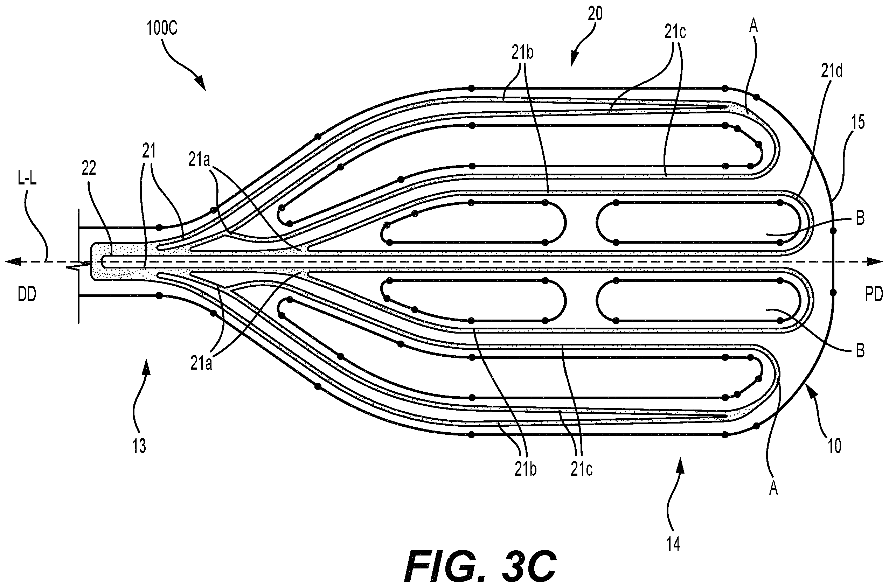

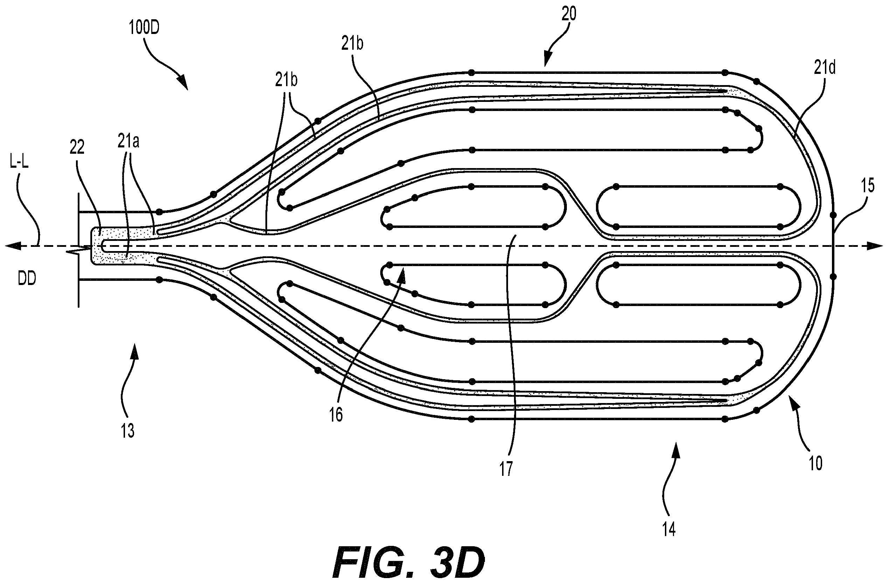

The example shown in FIG. 3A shows a framework 20 in which each side member 21 includes a proximal portion that divides into three curvilinear members 21a extending along the longitudinal axis L-L to rejoin proximate the distal end 15 of the flexible circuit layer 10. It is noted that FIGS. 3A, 3B, 3C and 3D indicates "10" as the flexible circuit layer for convenience and in practical terms, element "10" can represent just the polymer layer 30 alone as well as both the flexible circuit layer 10 and the polymer layer 30 in a complete assembly. FIG. 3B shows a framework 20 in which each side member 21 includes a proximal portion that divides into four curvilinear members 21b extending along the longitudinal axis L-L to rejoin proximate the distal end 15 of the flexible circuit layer 10.

In some examples, such as that shown in FIG. 3C, the framework 20 can include a proximal end 22 that divides into ten side members 21a extending along the longitudinal axis L-L toward the distal portion 14 of the flexible circuit layer 10 so that a single proximal end of framework defines four main fingers or loops with each outer loop A having three curvilinear members and inner loops B, each having two curvilinear members. In some examples, two side members 21 on one side of the longitudinal axis L-L can include an outer side member 21b and an inner side member 21c, the outer side member 21b divides into three curvilinear members 21b extending along the longitudinal axis L-L to rejoin proximate the distal end 15 of the flexible circuit layer 10 and the inner side member 21d divides into two curvilinear members 21b extending along the longitudinal axis L-L to rejoin proximate the distal end 15 of the flexible circuit layer 10 to form a loop for each "thumb" portion of the two thumbs or loops.

FIG. 3D shows a framework 20 that includes a proximal end 22 that divides into two symmetrical side members 21a extending along the longitudinal axis L-L so that no framework 20 is disposed on an area 16 over the longitudinal axis L-L from the proximal portion 13 to a central portion 17 of the flexible circuit layer 10. That is, the framework 20 defines two symmetrical loops akin to two "thumbs" opposing each other. In some examples, the two symmetrical side members 21a can be spaced apart approximately the diameter of a circular electrode 41a, 41b such that an electrode pair 40 can be disposed therebetween.

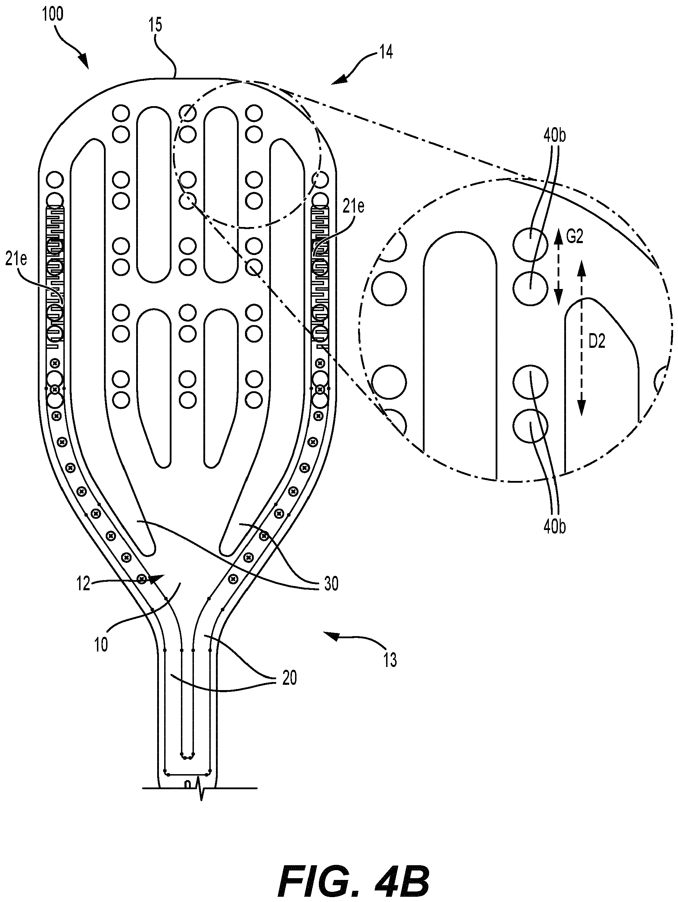

The example illustrated in FIG. 4A and FIG. 4B shows the first side 11 and the second side 12 of a flexible circuit layer 10 of an end effector having a framework 20 which includes a proximal end 22 that divides into a plurality of meandering members 21e extending along the longitudinal axis L-L towards the distal portion 14 of the flexible circuit layer 10.

In some examples, the meandering members 21e can include a sinusoidal member 21e extending along the longitudinal axis L-L on a plane. The meandering members 21e illustrated in FIG. 4A serve as representative examples of structures also contemplated herein which when applied to the framework 20 serve to modify the ability of the framework 20 and thus the end effector 100 to flex with respect to various directions and with respect to portions of the end effector 100. For example, the meandering members 21e can define a sinusoidal path wherein the wavelength of the sinusoid increases from the proximal portion 13 to the distal portion 14. Additionally, the meandering members 21e can define a sinusoidal path wherein the wavelength of the sinusoid decreases from the proximal portion 13 to the distal portion 14, or the meandering members 21e can define a sinusoidal path for only part of the total length of the frame member 21.

Returning to examples of electrode layouts - FIG. 4A shows a first side 11 of a flexible circuit layer in an end effector 100. This example includes a plurality of pairs of first electrodes 40a disposed on the first surface 11, the electrodes of each pair of first electrodes 40a being spaced apart over a minimum gap G₁ and each pair of first electrodes 40a spaced apart from adjacent pairs of first electrodes 40a over a predetermined distance Di.

FIG. 4B shows a second side 12 of a flexible circuit layer 10 in an end effector 100. This example includes a plurality of pairs of second electrodes 40b disposed on the second surface 12, the electrodes of each pair of second electrodes 40b being spaced apart over a minimum gap G₂ and each pair of second electrodes 40b spaced apart from adjacent pairs of second electrodes 40b over a predetermined distance D₂.

In some examples, there are about 92 electrodes 40 and about 46 pairs of first 40a and second 40b electrodes. Details of the spacing of the electrodes for each pair vs spacing between discrete sets of pairs of electrodes can be found in US Provisional Patent Application S.N. 63/406,673 (Attorney Docket No. BIO6749USPSP3) filed on September 14, 2022 and included in the Appendix included in priority application U.S. 63/478,059.

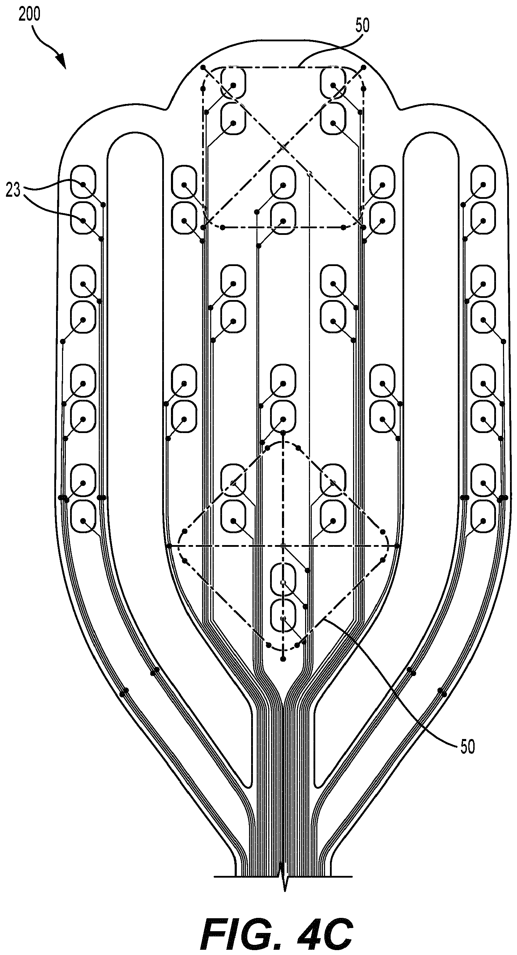

FIG. 4C shows an example end effector 100 wherein the electrodes 40 are oblong and each is aligned coaxially with a terminal ramification point 23 of the framework 20, this example having a ramified framework 20 with a density of terminal ramification points that increases from the proximal end 13 to the distal end 14. Terminal ramification points 23 are the points at which a branch of the framework terminates and past which a branch of the framework 20 does not further bifurcate. The points at which members 21 of framework bifurcate can be rounded, and the flat profile of some examples of framework 20 is conducive to laser cutting.

The distal end 15 of the flexible circuit layer 10 in FIG. 4C is elongated and bulbous in comparison to that in FIG. 4B, and this can allow for placement of more electrodes 40 along the longitudinal axis L-L, particularly when using the oblong electrodes 40 shown therein.

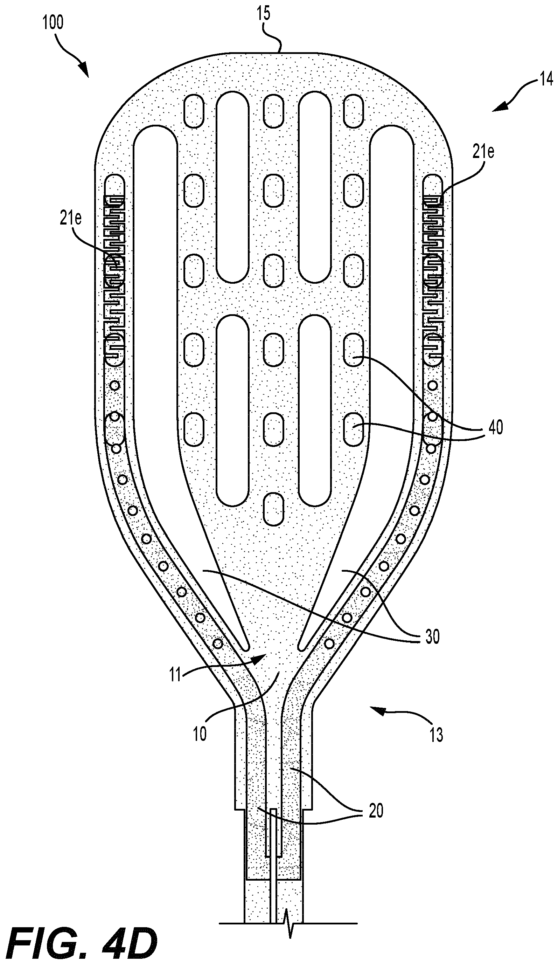

The example end effector 100 shown in FIG. 4D shows an example end effector with meandering members 21e similar to those depicted in FIG. 4A and oblong electrodes similar to those depicted in FIG. 4C.

The framework 20 examples contemplated herein function cooperatively with the flexible circuit layer 10 and flexible polymer layer 20 of end effector 100 in order to increase the mapping accuracy of the catheter by allowing better contact of end effector 100 with the target anatomy.

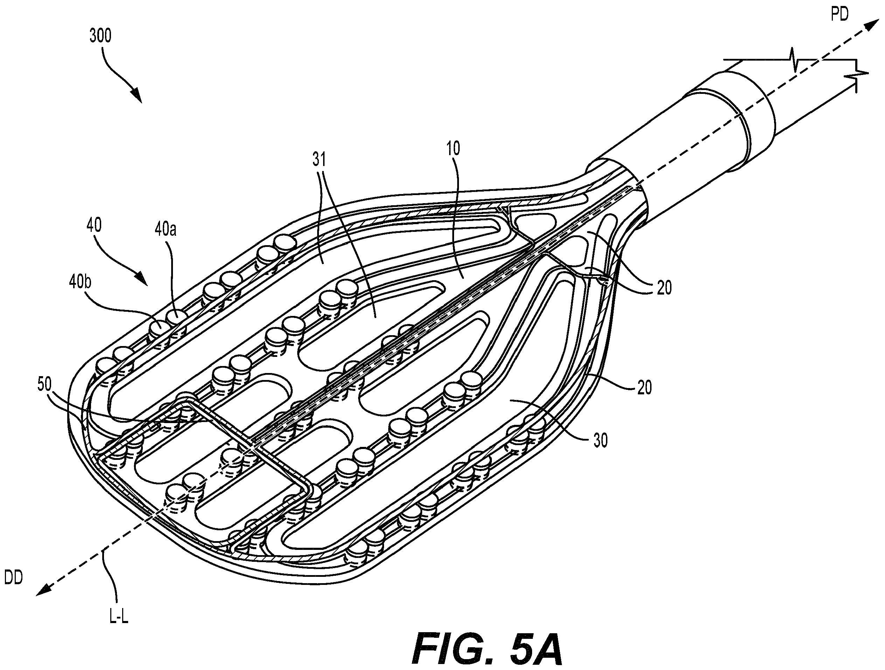

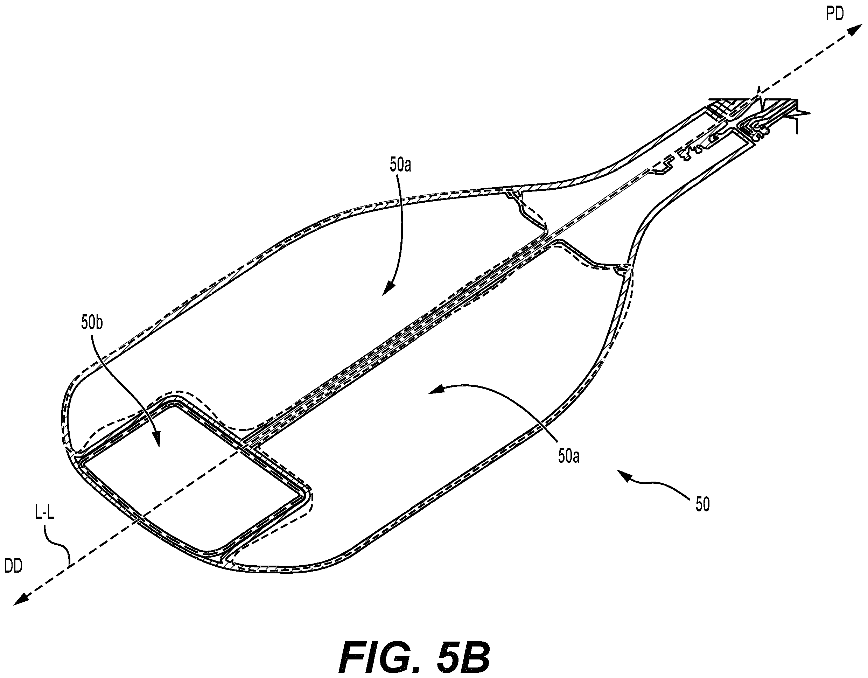

FIGS. 5A-5B show an end effector 100 including a plurality of coils 50 disposed on the flexible circuit layer 10. FIG. 5B shows only the coils 50 of the plurality of coils of the end effector 100, and FIG. 5A shows end effector 100. The plurality of coils 50 can include two elongate coils 50a, one on either side of the longitudinal axis and coplanar with the first surface and a rectangular coil 50b disposed proximate the distal portion of the flexible circuit and coplanar with the first surface. The flexible polymer layer 30 can have portions 31 lying inside of the coils 50 removed in order to make the end effector 100 more permissive to flexion while maintaining the geometrical integrity and sensing ability of the coils 50. Additionally, in some examples, the framework 20 can be shaped to make the end effector 100 more permissive to flexion while maintaining the geometrical integrity and sensing ability of the coils 50.

The coils 50 can be used in conjunction with one or more magnetic field generators (not shown) to determine the position of the end effector 100 within the subject via triangulation. Electromagnetic location sensing technique is described in US Patent Nos. 5,391,199; 5,443,489; 5,558,091; 6,172,499; 6,690,963; and 6,788,967 which are included in the Appendix included in priority application U.S. 63/478,059.

The above examples of the effector 100 can use the flexible circuit layer 10 and the framework 20 covered by the flexible polymer layer 30 to form a double-sided end effector without an intervening support layer. In lieu of mounting or coating electrodes 40 on a support layer, the flexible polymer layer 30 provides some structural rigidity as an outer overcoating.

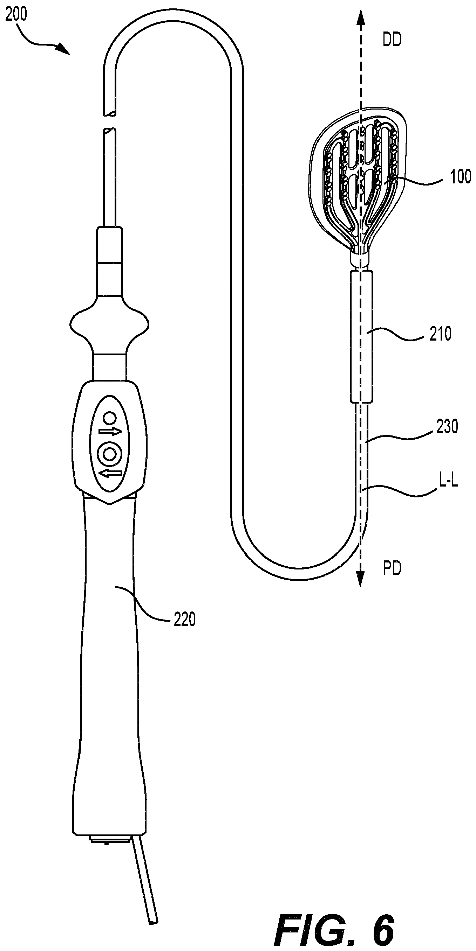

Reference is made to FIG. 1A showing an end effector for a catheter. The present disclosure provides a catheter assembly 200 as shown in FIG. 6 which can include a tubular member 230 extending along a longitudinal axis L-L and configured to deliver end effector 100 to an out of a sheath 210. A physician can manipulate the catheter 200 with handle 220. Appropriate examples for catheter assembly 200 and its subcomponents such as handle 220, sheath 210, tubular member 230, and others not mentioned herein are described in US Patent Application No. 17/029,752 published as US-2021-0369339-A1, which is included in the Appendix included in priority application U.S. 63/478,059.

The disclosed technology described herein can be further understood according to the following clauses:
Clause 1: An end effector for a catheter, the end effector comprising: a flexible circuit layer extending along a longitudinal axis from a proximal portion to a distal portion, the flexible circuit layer comprising a first surface and a second surface opposite the first surface; a framework extending generally parallel to the flexible circuit layer along the longitudinal axis from the proximal to the distal portion; and a flexible polymer layer encapsulating both the framework and the flexible circuit layer.
Clause 2: The end effector of clause 1, further comprising one or more first electrodes affixed to the first surface of the flexible circuit layer with its contact surface exposed through the flexible polymer layer to the ambient environment.
Clause 3: The end effector of clause 2, further comprising one or more second electrodes affixed to the second surface of the flexible circuit layer with its contact surface exposed through the flexible polymer layer to the ambient environment.
Clause 4: The end effector of clause 3, in which the first electrodes are axially aligned with the second electrodes to define pairs of opposite facing electrodes.
Clause 5: The end effector of clause 4, in which pairs of opposite facing electrodes are aligned generally parallel to the longitudinal axis.
Clause 6: The end effector of clause 4, in which pairs of opposite facing electrodes are aligned generally transverse to the longitudinal axis.
Clause 7: The end effector of clause 2, further comprising a plurality of pairs of first electrodes disposed on the first surface, the electrodes of each pair of first electrodes being spaced apart over a minimum gap and each pair of first electrodes spaced apart from adjacent pairs of first electrodes over a predetermined distance.
Clause 8: The end effector of clause 7, further comprising a plurality of pairs of second electrodes disposed on the second surface, the electrodes of each pair of second electrodes being spaced apart over a minimum gap and each pair of second electrodes spaced apart from adjacent pairs of second electrodes over a predetermined distance.
Clause 9: The end effector of clause 1, in which the framework comprises a proximal end that divides into two symmetrical side members extending along the longitudinal axis so that no framework is disposed on an area over the longitudinal axis from the proximal portion through the distal portion of the flexible circuit layer.
Clause 10: The end effector of clause 9, in which each side member comprises a proximal portion that divides into three curvilinear members extending along the longitudinal axis to rejoin proximate the distal end of the flexible circuit layer.
Clause 11: The end effector of clause 9, in which each side member comprises a proximal portion that divides into four curvilinear members extending along the longitudinal axis to rejoin proximate the distal end of the flexible circuit layer.
Clause 12: The end effector of clause 1, in which the framework comprises a proximal portion that divides into five side members extending along the longitudinal axis toward the distal portion of the flexible circuit layer.
Clause 13: The end effector of clause 12, in which two side members on one side of the longitudinal axis comprises an outer side member and an inner side member, the outer side member divides into three curvilinear members extending along the longitudinal axis to rejoin proximate the distal end of the flexible circuit layer and the inner side member divides into two curvilinear members extending along the longitudinal axis to rejoin proximate the distal end of the flexible circuit layer.
Clause 14: The end effector of clause 1, in which the framework comprises a proximal end that divides into two symmetrical side members extending along the longitudinal axis so that no framework is disposed on an area over the longitudinal axis from a proximal portion to a central portion of the flexible circuit layer.
Clause 15: The end effector of clause 1, in which the framework comprises a proximal portion that divides into a plurality of meandering members extending along the longitudinal axis towards the distal portion of the flexible circuit layer.
Clause 16: The end effector of clause 15, in which the meandering members comprise a sinusoidal member extending along the longitudinal axis on a plane.
Clause 17: The end effector of clause 1, further comprising a plurality of coils disposed on the flexible circuit layer.
Clause 18: The end effector of clause 1, in which the flexible circuit layer defines a planar configuration extending through the longitudinal axis with the framework disposed inside the flexible circuit layer.
Clause 19: The end effector of clause 1, further comprising a plurality of coils disposed on the flexible circuit layer.
Clause 20: The end effector of clause 1, in which the flexible circuit layer defines a planar configuration extending through the longitudinal axis with the framework disposed inside the flexible circuit layer.
Clause 21: The end effector of clause 17, the plurality of coils comprising: an elongate coil on either side of the longitudinal axis and coplanar with the first surface; and a rectangular coil disposed proximate the distal portion of the flexible circuit and coplanar with the first surface.
Clause 22: The end effector of clause 4, wherein each of the electrode pairs is aligned coaxially with a terminal ramification point of the framework.

## Claims

1. An end effector for a catheter, the end effector comprising:
a flexible circuit layer extending along a longitudinal axis from a proximal portion to a distal portion, the flexible circuit layer comprising a first surface and a second surface opposite the first surface;
a framework extending generally parallel to the flexible circuit layer along the longitudinal axis from the proximal to the distal portion; and
a flexible polymer layer encapsulating both the framework and the flexible circuit layer.

2. The end effector of claim 1, further comprising one or more first electrodes affixed to the first surface of the flexible circuit layer with its contact surface exposed through the flexible polymer layer to the ambient environment.

3. The end effector of claim 2, further comprising one or more second electrodes affixed to the second surface of the flexible circuit layer with its contact surface exposed through the flexible polymer layer to the ambient environment.

4. The end effector of claim 3, in which the first electrodes are axially aligned with the second electrodes to define pairs of opposite facing electrodes.

5. The end effector of claim 4, in which pairs of opposite facing electrodes are aligned generally parallel to the longitudinal axis, or generally transverse to the longitudinal axis.

6. The end effector of claim 1, further comprising a plurality of pairs of first electrodes disposed on the first surface, the electrodes of each pair of first electrodes being spaced apart over a minimum gap and each pair of first electrodes spaced apart from adjacent pairs of first electrodes over a predetermined distance, optionally further comprising a plurality of pairs of second electrodes disposed on the second surface, the electrodes of each pair of second electrodes being spaced apart over a minimum gap and each pair of second electrodes spaced apart from adjacent pairs of second electrodes over a predetermined distance.

7. The end effector of claim 1, in which the framework is coupled to the flexible circuit layer, the framework comprises a proximal end that divides into two symmetrical side members extending along the longitudinal axis so that no framework is disposed on an area over the longitudinal axis from the proximal portion through the distal portion of the flexible circuit layer.

8. The end effector of claim 7, in which the end effector remains in contact against a generally planar surface with a force applied to the proximal portion of the end effector in a vertical axis with respect to the generally planar surface, optionally in which the end effector remains in contact against a generally planar surface with a force applied to the proximal portion of the end effector in a vertical axis with respect to the generally planar surface and a force applied generally parallel to the generally planar surface.

9. The end effector of claim 7, in which each side member comprises a proximal portion that divides into three curvilinear members extending along the longitudinal axis to rejoin proximate the distal end of the flexible circuit layer, or into four curvilinear members extending along the longitudinal axis to rejoin proximate the distal end of the flexible circuit layer.

10. The end effector of claim 1, in which the framework comprises a proximal portion that divides into five side members extending along the longitudinal axis toward the distal portion of the flexible circuit layer.

11. The end effector of claim 10, in which two side members on one side of the longitudinal axis comprise an outer side member and an inner side member, the outer side member divides into three curvilinear members extending along the longitudinal axis to rejoin proximate the distal end of the flexible circuit layer and the inner side member divides into two curvilinear members extending along the longitudinal axis to rejoin proximate the distal end of the flexible circuit layer.

12. The end effector of claim 1, in which the framework comprises a proximal end that divides into two symmetrical side members extending along the longitudinal axis so that no framework is disposed on an area over the longitudinal axis from the proximal portion to a central portion of the flexible circuit layer.

13. The end effector of claim 1, in which the framework comprises a proximal portion that divides into a plurality of meandering members extending along the longitudinal axis towards the distal portion of the flexible circuit layer, optionally in which the meandering members comprise a sinusoidal member extending along the longitudinal axis on a plane.

14. The end effector of claim 1, further comprising a plurality of coils disposed on the flexible circuit layer.

15. The end effector of claim 14, the plurality of coils comprising:
an elongate coil on either side of the longitudinal axis and coplanar with the first surface; and
a rectangular coil disposed proximate the distal portion of the flexible circuit and coplanar with the first surface.
